# EUROPEAN PATENT SPECIFICATION

(11) **EP 0 758 646 B1**
(45) Date of publication and mention of the grant of the patent: **26.04.2000**
(21) Application number: 96420267.5
(22) Date of filing: 05.08.1996
(51) Int. Cl.: C07D 231/08, G03C 5/30

(54) **Compounds of the 3-pyrazolidone type useful in photography**
In der Photographie verwendbare Verbindungen des 3-Pyrazolidon-Typs
Composés du type 3-pyrazolidone utiles en photographie

(30) Priority: 10.08.1995 FR 9509850
(43) Date of publication of application: 19.02.1997
(73) Proprietor: EASTMAN KODAK COMPANY, Rochester, New York 14650-2201 (US)
(72) Inventor: Roussilhe, Jacques, c/o Kodak-Pathe, 71102 Chalon sur Saone Cedex (FR); Tsoi, Siu-Chung, Harrow, Middlesex HA1 4TY (GB)
(74) Representative: Parent, Yves

(56) References cited:
- GB-A- 1 025 575
- US-A- 2 743 279
- US-A- 4 266 002
- US-A- 5 236 816
- CHEMICAL ABSTRACTS, vol. 73, no. 6, 10 August 1970 Columbus, Ohio, US; abstract no. 30672v, V.L. ABRITALIN ET AL.: "Developer for light-sensitive silver halide photographic materials." page 386; column 1; XP002017173 & SU-A-265 715 (V.L. ABRITALIN ET AL.) 9 March 1970

## Description

The present invention concerns photography and novel compounds of the 3-pyrazolidone type which can be used for the development of silver halide photographic products, in particular as co-developers for developing black and white photographic papers or films.

Developing agents are described Chimie et Physique Photographiques, P Glafkides, Chapter IX, pages 152-170, fifth edition. In general, a principal developing agent is used in association with an auxiliary developing agent. A synergy effect is observed between the principal developing agent, hereinafter referred to as the "developing agent", and the auxiliary developing agent or "co-developing agent" when the combined activity of the mixture of these two agents is greater than the sum of the activities of each of these agents used separately in the same solution. This phenomena, referred to as "superadditivity" is explained in Mason "Photographic Processing Chemistry", Focal Press, London, 1975.

Di- and polyhydroxyphenols, for example hydroquinone, and reductones, for example compounds of the ascorbic acid type, are the developing agents which are most used in practice in black and white developing solutions.

Amongst the most often used co-developing agents are aminophenols, such as Elon® (methyl-p-aminophenol sulphate), 1-phenyl-3-pyrazolidones or phenidones, such as phenidone-A (1-phenyl-3 pyrazolidone), phenidone-B (1-phenyl-4 methyl-3-pyrazolidone), dimezone (1-phenyl-4-4'-dimethyl-3-pyrazolidone) or dimezone-S (1-phenyl-4-methyl-4'-hydroxymethyl-3-pyrazolidone). Additional representative examples of aminophenols and phenidones are described in US patents 2 688 549, 2 691 589, 3 865 591, 4 269 929, 4 840 879 and 5 236 816, and in the article by G E Ficken and B G Sanderson, The Journal of Photographic Science, Vol 11, 1963, pages 157-164.

These co-developers have low solubility in water, which presents drawbacks with regard to the manufacture of the developer and is ease of use. In addition, photographic processing solutions are often in the form of powders to be dissolved in water or liquid concentrates to be diluted before use. These powders must be easy to solubilise and the co-developers must be sufficiently soluble to enable concentrates to be formulated.

Patent application EP-A 528 480 describes a radiographic product comprising a 3-pyrazolidone substituted by a carboxy group directly attached to the phenyl ring. This compound is used as an anti-fogging agent. The radiographic product is developed with a conventional developer comprising hydroquinone and a 1-phenyl-pyrazolidone-1-one co-developer.

The problem of the low solubility of phenidone or dimezone-S was resolved in US patent 4 753 869 by preparing these 1-phenyl-3-pyrazolidones in the form of salts of four particular acids including sulpho groups which dissolve easily in water and are stable during storage. The activity of these compounds in combination with hydroquinone is said to be comparable with that of pyrazolidones which are not in the form of salts.

The article in Zhurnal Nauchnoi i Prikladnoi Fotografii i kinematografii 10, (5), 321-329 (1963) by V L Abritalin et al describes photographic developers comprising hydroquinone and a large number of derivatives of 3-pyroazolidones, some of which carry solubilising groups on the benzene ring. It is shown in this article that the introduction of the carboxy or sulpho solubilising groups on the benzene ring brings a significant reduction in superadditivity. This tendency is also noted by G E Ficken and B G Sanderson in The Journal of Photographic Science, Vol 11, 1963, pages 157-160, who report that the introduction of a carboxylic group on the phenidone reduces the superadditivity between phenidone and hydroquinone.

Hydroquinone-based developers generally give good results but present drawbacks with regard to health and the environment. This is why ascorbic acid is used in place of hydroquinone in association with phenidones in developing compositions described in many patents.

Amongst them, US patent 5 098 819 describes a developing composition comprising ascorbic acid or its derivatives, and a 3-pyrazolidone compound. The developer in the examples contains sodium erythorbate, phenidone or dimezone-S and potassium carbonate. This developing composition is less toxic than those containing hydroquinone and caustic bases and the sensitometric results are close to those obtained with compositions containing hydroquinone.

US patent 3 938 997 describes a developing solution for the fast development of high-contrast products of the microfilm type. These solutions comprise three developing agents : the first is a ferrous chelate, the second is a compound of the ascorbic acid type, the third is phenidone, Glycine®, hydroxylamine sulphate, etc. Developing solutions are obtained which can be easily concentrated.

Patent application EP-A 588 408 describes a developer comprising a principal developing agent of the ascorbic acid type and a mixture of two co-developing agents of the phenidone type selected from phenidone-A, phenidone-B and dimezone, dimezone-S. The developing composition makes it possible to obtain an improved sensitometric stability which does not depend on the reduction in the pH observed during continuous processing without regeneration. The examples concern emulsions for microfilms.

US patent 5 264 323 described compositions for the development of films for graphic arts comprising ascorbic acid or its isomers and a 3-pyrazolidone or aminophenol compound.

Patent application EP-A 461 783 describes a developing composition comprising ascorbic acid or its derivatives, a 3-pyrazolidone compound, sulphite or bisulphite and sodium sulphate or glutaraldehyde, which can be used for the development of medical radiographs.

Patent application WO 95/00881 describes stable developers comprising a compound of the ascorbic acid type or a sugar-type derivative of this acid, and a co-developing agent selected from phenidones and aminophenols (or a mixture of these) for the fast development of graphic arts films incorporating compounds of the hydrazine type.

US patent 5 384 232 describes developing compositions comprising sodium erythorbate or erythorbic acid associated with Dimezone-S for developing lithographic films.

Patent application EP-A 603 586 describes formulations of concentrates for developers incorporating a compound of the ascorbic acid type and a co-developer, preferably Dimezone-S.

Thus one of the objects of the present invention is to obtain novel developers containing compounds of the 3-pyrazolidone type which, when they are used as co-developing agents, have improved solubility without the superadditivity effect being degraded.

Another object of the invention is to obtain novel developing compositions comprising a developing agent for silver halides selected from reductones or compounds of the hydroquinone type and at least one co-developing agent which is one of the compounds of the 3-pyrazolidone type according to the invention.

The preferred developing compositions comprise a developing agent selected from ascorbic acid, sugar-type derivatives of ascorbic acid, stereoisomers, diastereoisomers, precursors of these acids and their salts and at least one co-developing agent which is one of the compounds of the 3-pyrazolidone type according to the invention.

In the following description, reference will be made to the following figures:

Figure 1, which shows the sensitometric curves obtained with reference developers and with developers according to the invention comprising ascorbic acid and compounds II or III according to the invention in a "slow access" process for the development of a low-contrast black and white photographic film, or "film A".

Figure 2, which shows the sensitometric curves obtained with developers according to the invention comprising ascorbic acid, its isomers, derivatives or precursors and compound II in a "slow access" process for the development of film A.

Figure 3, which shows the sensitometric curves obtained with a reference developer and with a developer according to the invention comprising ascorbic acid and compound II, III or IV according to the invention in a "rapid access" process for the development of a film for medical radiography, or "film B".

Figure 4, which shows the sensitometric curves obtained with a reference developer and with a developer according to the invention comprising ascorbic acid and compound II in a "rapid access" machine development process for the development of film B.

Figure 5, which shows the sensitometric curves obtained with a reference developer and with a developer according to the invention comprising hydroquinone and compounds II or III in a "slow access" process for the development of film A®.

Figure 6, which shows the sensitometric curves obtained with a reference developer and with a developer according to the invention comprising hydroquinone and, as a co-developer, compounds II and III in a "rapid access" process for the development of a film for medical radiography or "film C".

Black and white photographic products can be considered to form two distinct groups according to the development time. Thus black and white films for photography and cinema, industrial radiographs and black and white papers are developed relatively slowly. Typical development times are around 1 to 3 minutes for papers and 4 to 12 minutes for films. The development temperature is between 18 and 27°C but can also be higher. This is what is referred to in the art as a long access time or "slow access" type. A known developer of the "slow access" type is the Kodak D-76® powder universal developer used for example for the development of black and white photographic films and which comprises hydroquinone and Elon®.

Rapid development systems, also referred to as "short access time" or "rapid access" types, are used for the development of medical radiographs, graphic arts and microfilms. These products are developed with highly active solutions. The development time is around 30 seconds or less and the development temperature is approximately 35°C. An example of a developer of the "rapid access" type is the Kodak RP X-OMAT® developer, used for the development of films for medical radiography, which comprises hydroquinone and phenidone-A as a co-developer. Other "rapid access" developers comprising ascorbic acid and dimezone-S as a co-developer are described in Research Disclosure of August 1993, Article 35249.

The compounds of the 3-pyrazolidone type according to the invention are 3-pyrazolidones which have solubilising groups which are not directly attached to the phenyl ring or to the pyrazolidino ring and comply with the general formula: 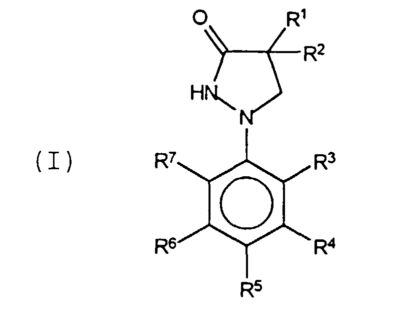 where R¹ and R² individually represent, alkyl substituted alkyl, or a group represented by the formula:

(CH₂)ₘ― (L)ₙ ― A ― (Sol)

where
m is between 0 and 5 and n is 0 or 1,
L represents a divalent group selected from 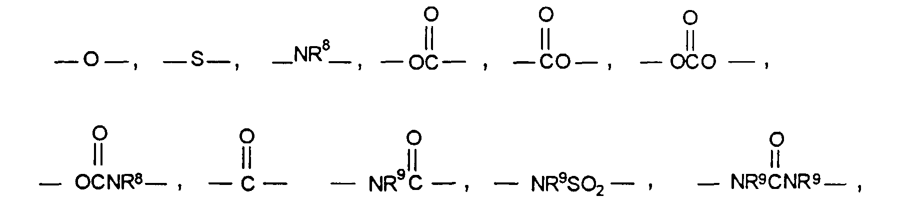 where R⁸ = R⁹ or A-(Sol), R⁹ = H, alkyl or aryl;
A represents a divalent group selected from 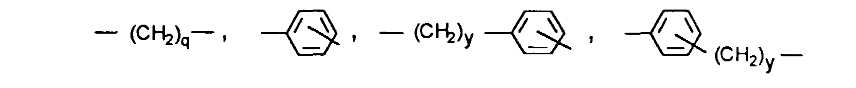 where q is between 0 and 5, and y is between 1 and 3;
   (Sol) is a solubilising group selected from:
   CO₂H, SO₃H, NHSO₂R¹⁰, SO₂NH₂, SO₂NHR¹⁰ polyhydroxyalkyl, 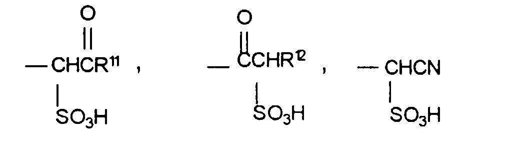 where R¹⁰ is alkyl or aryl, R¹¹ is OH, alkyl or aryl and R¹² is hydrogen, alkyl or aryl;
R³ to R⁷ in formula (I) individually represent hydrogen, alkyl, substituted alkyl, alkoxy, substituted alkoxy, aryloxy, substituted aryloxy, or a group represented by the formula:

   (X)ₚ ― (CH₂)ₘ ― (L)ₙ A ― (Sol)

   where p = 0 or 1;
X represents a divalent group selected from ― O― , ― S ― , ―NR⁸―
   m, L, n, A, (Sol) and R⁸ are as defined previously
   with the additional proviso that at least one of the radicals R¹ to R⁷ must contain a group (Sol) and that (sol) is not directly attached to the phenyl ring or to the pyrazolidone ring.

Examples of compounds according to the invention comprising a solubilising group indirectly attached to the pyrazolidino ring are:
(4-methyl-3-oxo-1-phenylpyrazolidin-4-yl)methyl 2-sulphobenzoate (Compound II) 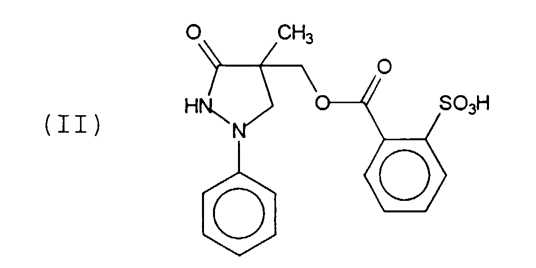
{1-(3,4-dimethylphenyl)-4-methyl-3-oxo-pryazolidin-4-yl}methyl 2-sulphobenzoate (Compound III) 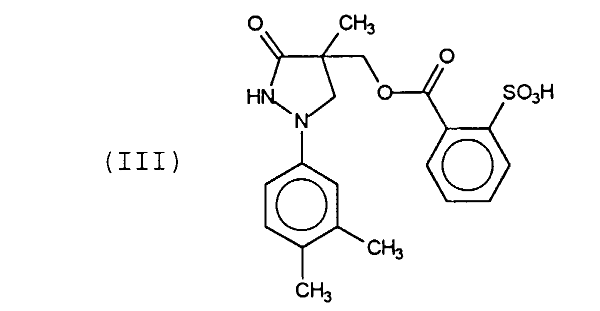
{1-(3,4-dimethoxyphenyl)-4-methyl-3-oxo-pyrazolidin-4-yl}methyl 2-sulphobenzoate (Compound IV)

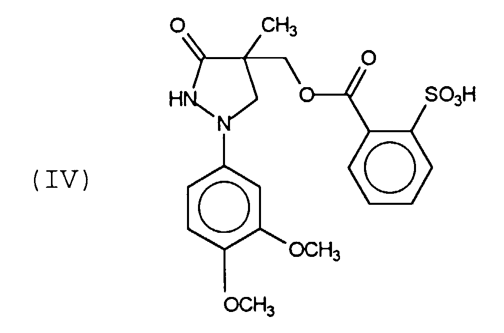

The invention also concerns developing solutions comprising a developer for silver halides selected from di- and poly- hydroxybenzenes and reductones and at least one co-developer which is one of the novel compounds of the 3-pyrazolidone type as defined previously.

The compound according to the invention can be used as the sole co-developer or else in mixture with other compounds according to the invention or with known aminophenols or phenidones, such as Elon®, Phenidone-A, Phenidone-B, Dimezone, Dimezone-S or 4,4-bis(hydroxymethyl)-1-phenyl-3-pyrazolidinone. In general terms, developing agents which can be used are described in Research Disclosure, publication 36544, September 1994, Chapter XIX, page 536.

In practice, use is made of a quantity of co-developer of the 3-pyrazolidone type in the developing composition of between 0.0005 and 0.2 mol/l, and preferably between 0.001 and 0.006 moles/litre of ready-to-use solution.

The novel compounds of the 3-pyrazolidone type according to the invention can be used in developing compositions as co-developers for "slow access" or "rapid access" in combination with all known developers.

In black and white developing solutions, the developer is a compound preferably selected from di- and polyhydroxybenzenes and reductones. The dihydroxybenzenes most often used are hydroquinone, catechol and derivatives thereof. Examples of reductones include ene-diols, ene-aminols, ene-diamines, thio-enols and enamine-thiols. The reductones most often used are cited in US patent 2 691 589, in particular ascorbic acids, its stereoisomers, diastereoisomers and sugar-type derivatives.

Hydroquinone can be used as a developer in the developing compositions according to the invention but, in order to preserve the environment, ascorbic acid, its sugar-type derivatives, stereoisomers, diastereoisomers, precursors of these acids and their salts are preferred to this.

For example, use can be made as a developer of D-isoascorbic (or erythorbic) acid, L-ascorbic acid and their salts such as sodium or potassium ascorbate or erythorbate; sugar-type derivatives of ascorbic acid, for example D-glucoascorbic acid, 6-desoxy-1-ascorbic acid, L-rhamnoascorbic acid, L-fucoascorbic acid, D-glucoheptoascorbic acid, sorboascorbic acid, ω-lactoascorbic acid, maltoascorbic acid, L-araboascorbic acid, L-glucoascorbic acid, D-galactoascorbic acid, L-guloascorbic acid and L-alloascorbic acid, and imino-L-ascorbic acid; cetal derivatives of L-ascorbic and D-isoascorbic acids, for example 5,6-isopropylene ascorbic acid; and ascorbic acid precursors, for example methyl-2-cetogluconate or a mixture of these.

In practice, a quantity of developer of the hydroquinone or ascorbic acid type in the developing solution of between 0.05 and 0.4 mol/l and preferably between 0.15 and 0.30 moles/l of ready-to use solution is used.

The developers according to the invention may contain, in addition to the developer and co-developer, many conventional additives such as solvents for silver halides, alkaline bases, organic or inorganic anti-fogging agents, pH buffering agents, antioxidants, sequestering agents, agents for controlling swelling, hardening agents and wetting agents. Within the scope of the invention, the developing composition is open to many variants accessible to experts according to the application envisaged. These developers can be concentrated in liquid form or in the form of powders which must be dissolved in water in order to obtain the ready-to-use solution.

The compounds according to the invention have improved solubility compared with known phenidones. Surprisingly, the presence of these solubilising groups which are not directly attached to the phenyl ring or to the pyrazolidino ring do not cause the appreciable reduction in superadditivity observed in the article in Zhurnal Nauchnoi i Prikladnoi Fotografii i kinematografii already cited. On the contrary, the developing solutions comprising these compounds as co-developers have a satisfactory photographic activity.

These developing solutions according to the invention are useful for developing black and white products, such as products for the graphic arts, radiographic products, black and white photographic films and papers or microfilms or for the black and white development stage of colour reversible films and papers.

In addition the developing solutions according to the invention containing a developer of the ascorbic acid type have little tendency to form silver sludge or to cause the deposition of metallic silver on the equipment.

The invention is illustrated by the following examples:

### EXAMPLES

### Example 1

### Synthesis of the compound (4-methyl-3-oxo-1-phenylpyrazolidin-4-yl)methyl 2-sulphobenzoate (Compound II)

To a suspension of 4-methyl-4-hydroxymethyl-1-phenyl-3-pyrazolidinone (dimezone-S) (10 g, 48.5 mmol) in dry acetonitrile (200 ml), was added 2-sulphobenzoic acid cyclic anhydride (8.9 g, 48.5 mmol) in a single portion at room temperature with stirring. The reaction mixture was heated to reflux under nitrogen until complete dissolution was observed. The reflux was continued for 24 hours and the mixture was cooled in an ice/water bath for 2 hours. A solid precipitate was obtained which was recovered by filtration and washed with ice cooled acetonitrile. After drying under vacuum, 17.9 g (95%) of 4-methyl-3-oxo-1-phenyl-pyrazolidin-4-yl)methyl 2-sulphobenzoate (Compound II) were isolated as an off white solid.

### Example 2

### Synthesis of the compound {1-(3,4-dimethylphenyl)-4-methyl-3-oxo-pyrazolidin-4-yl}methyl 2-sulphobenzoate (Compound III)

To a suspension of {1-(3,4-dimethylphenyl)-4-hydroxymethyl-4-methyl-3-pyrazolidinone (10.0 g, 42.74 mmol) in dry acetonitrile (200 ml), was added 2-sulphobenzoic acid cyclic anhydride (7.86 g, 42.74 mmol) in a single portion at room temperature with stirring. The reaction mixture was heated to reflux under nitrogen for 22 hours until complete dissolution was observed. After cooling the mixture to room temperature, a heavy precipitate formed, which was recovered by filtration with suction. After washing with acetonitrile and drying under vacuum, 13.7 g (77%) of {1-(3,4-dimethylphenyl)-4-methyl-3-oxo-pyrazolidin-4-yl}methyl 2-sulphobenzoate (Compound III) were isolated in the form of a pale pink solid.

### Example 3

### Synthesis of the compound {1-(3,4-dimethoxyphenyl)-4-methyl-3-oxo-pyrazolidin-4-yl}methyl 2-sulphobenzoate (Compound IV)

To a suspension of {1-(3,4-dimethoxyphenyl)-4-hydroxymethyl-4-methyl-3-pyrazolidinone (1.0 g, 3.76 mmol) in dry tetrahydrofurane (20 ml) was added 2-sulphobenzoic acid cyclic anhydride (0.69 g, 3.76 mmol), in a single portion at room temperature with stirring. The reaction medium was heated to reflux under nitrogen for 24 hours until complete dissolution was observed. The mixture was cooled in a ice/water bath and the solid was recovered by filtration and washed with acetonitrile. After drying under vacuum, 0.93 g (55%) of {1-(3,4-dimethoxyphenyl)-4-methyl-3-oxo-pyrazolidin-4-yl}methyl 2-sulphobenzoate (Compound IV) was isolated in the form of a pale yellow solid.

### Example 4

### Slow access - Ascorbic acid developer - Co-developer Compound II or Compound III

In this example, film A was exposed at 2850 K with a colour correction filter for 1/25th of a second through a step tablet. This film was developed for 11 minutes at 23.5°C, was fixed for 5 minutes in T-MAX® fixer and was washed for 10 minutes under running water.

A developer solution according to the invention, comprising ascorbic acid as a developer and compound II or compound III as a co-developer, was assessed, comparing them with a Kodak D-76® powder universal developer which comprises hydroquinone and Elon® and with a second reference which comprises ascorbic acid and Elon®.

All the developers contained 45 mmol/l of developer, 12 mmol/l of co-developer and 126 g/l of potassium sulphite, and the pH was adjusted to 8.6 with potassium carbonate.

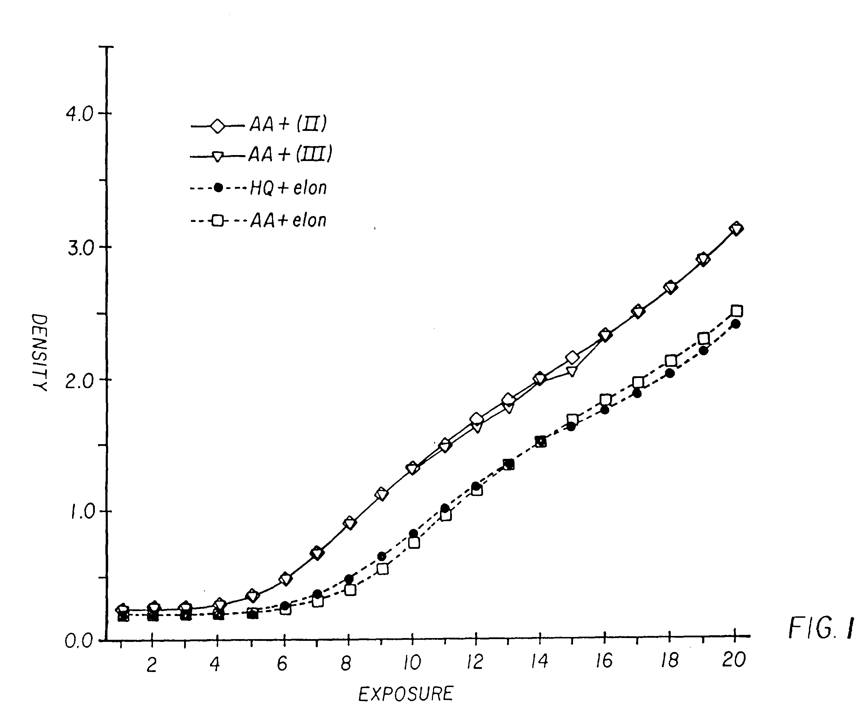

Figure 1 gives the sensitometric curves obtained with the two developers according to the invention and the reference developers. It can be seen that compounds II and III are more active than Elon® associated with ascorbic acid or hydroquinone.

### Example 5

### Slow access - Developers which are derivatives or precursors of ascorbic acid - Co-developer Compound II

Solutions of developers according to the invention comprising as a developer L-ascorbic acid, D-isoascorbic acid, methyl-2 cetogluconate and 5,6-isopropylidene ascorbic acid and comprising compound II as a co-developer, were evaluated under the same conditions as in Example 4.

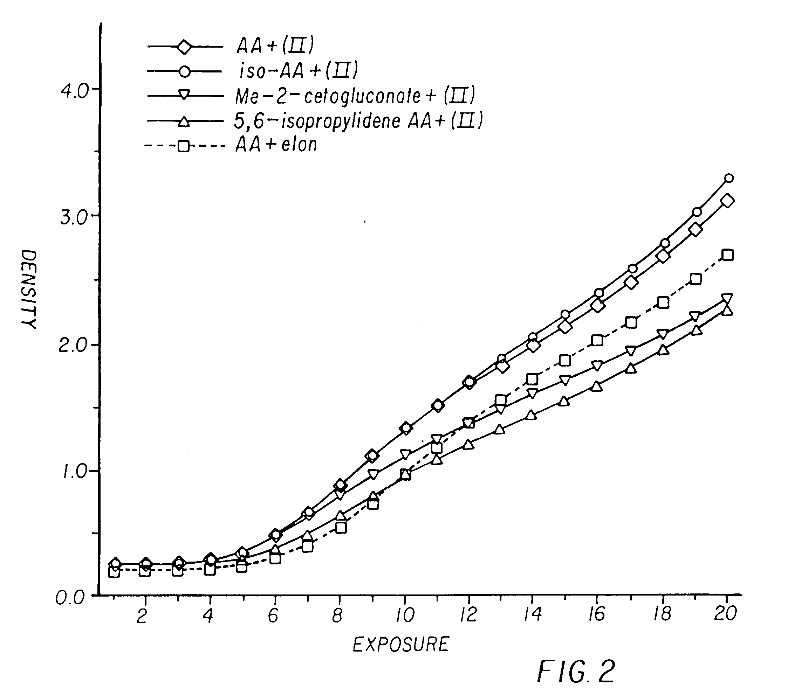

Figure 2 gives the sensitometric curves obtained for a development time of 11 minutes at 23.5°C.

It can be seen that acceptable sensitometric results were obtained with all the derivatives or precursors of ascorbic acid in combination with compound II. It will also be observed that D-isoascorbic acid behaves in substantially the same way as L-ascorbic acid.

### Example 6

### Rapid access - Ascorbic acid developer - Co-developer Compound II, III or IV

In this example, film B was exposed at 2850 K with a filter simulating the re-emission of a green screen for 1/50th of a second on both faces through a step tablet. This film was developed for 3 minutes at room temperature, manually reproducing the conditions of X-OMAT® processing machines, was fixed for 2 minutes and washed for 3 minutes in running water.

In this example the developer whose formula is given in Research Disclosure of August 1993, Article 35249 (already cited) was used as a reference:

| | |
|---|---|
| ascorbic acid | 32.0 g/l |
| dimezone-S (HMMP) | 2.5 g/l |
| Benzotriazole | 0.2 g/l |
| KBr | 4.0 g/l |
| K₂SO₃ | 50.0 g/l |
| K₂CO₃ | 100.0 g/l |
| Na DTPA* | 4.3 g/l |
| pH | 10.2 |

| | |
|---|---|
| *DTPA is diethylenetriaminopentacetic acid | |

In the compositions according to the invention, dimezone-S was replaced with an equimolar quantity of compound II, III or IV.

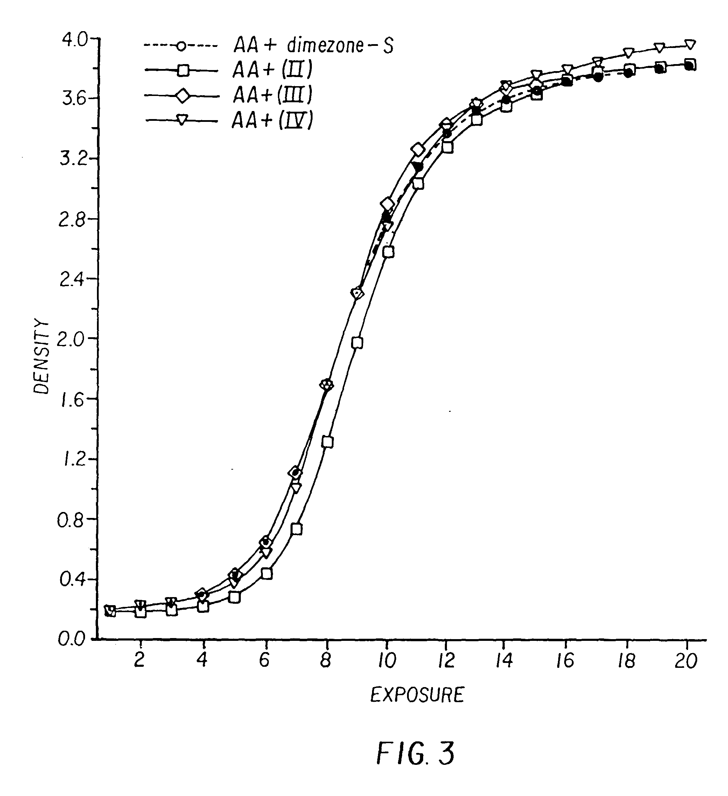

Figure 3 gives the sensitometric curves obtained. It can be seen that, in "rapid access", compounds II, III and IV combined with ascorbic acid give sensitometric results very close to those obtained with dimezone-S combined with ascorbic acid.

### Example 7

### Rapid access - Machine processing - Ascorbic acid developer - Co-developer compound II

In this example, film B was exposed at 2850 K with a filter simulating the re-emission of a green screen for 1/50th of a second on both faces through a step tablet. This film was developed for 25 seconds at 35°C in a KODAK X-OMAT® processing machine model ME-10.

In this example the reference developer of the previous example and a composition according to the invention containing ascorbic acid and compound II were used.

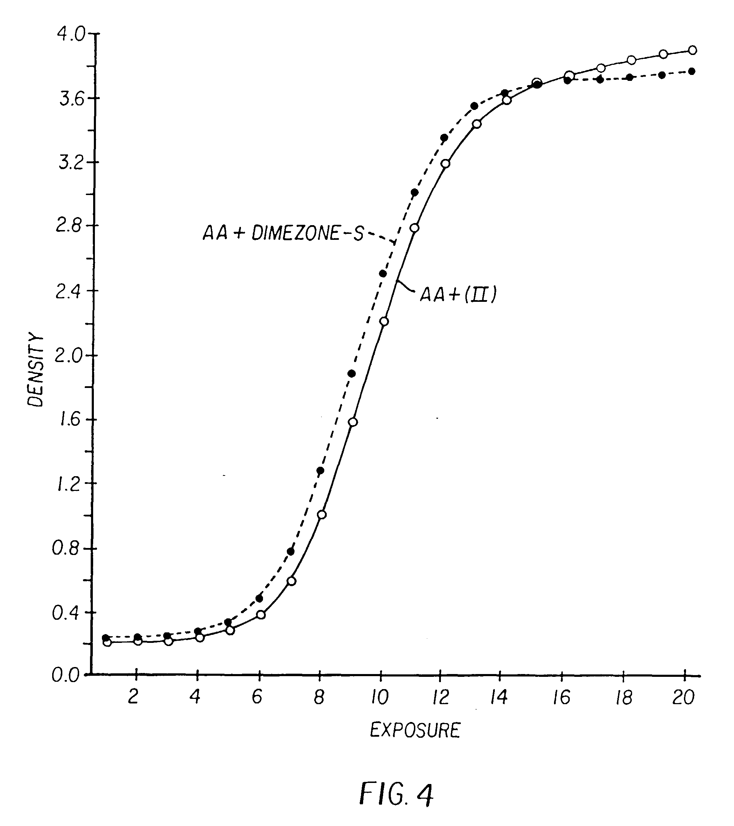

Figure 4 gives the sensitometric curves obtained. It will be observed that the results obtained in an automatic machine at 35°C satisfactorily reproduce the results of manual processing. In a machine, compound II combined with ascorbic acid gives, as in the previous example, sensitometric results very close to those obtained with dimezone-S combined with ascorbic acid.

### Example 8

### Slow access - Hydroquinone developer - Co-developer Compound II or Compound III

Solutions of developers according to the invention comprising hydroquinone as a developer and compound II or III as the co-developer were evaluated under the same conditions as in Example 4, comparing them with the Kodak D-76® developer, which comprises hydroquinone and Elon®.

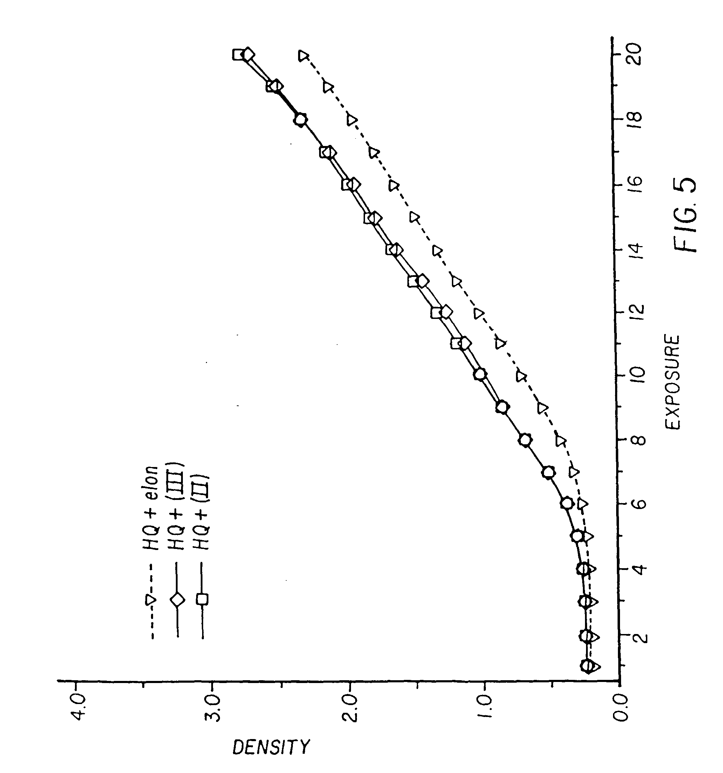

Figure 5 gives the sensitometric curves obtained for a development time of 11 minutes at 23.5°C. It can be seen that compounds II and III are more active than Elon® when they are combined with hydroquinone.

### Example 9

### Rapid access - Hydroquinone developer - Co-developer Compound II

In this example, film C was developed under the same conditions as in Example 6, using as a reference the RP X-OMAT® developer comprising hydroquinone and phenidone-A and two developers according to the invention comprising hydroquinone and compound II or III.

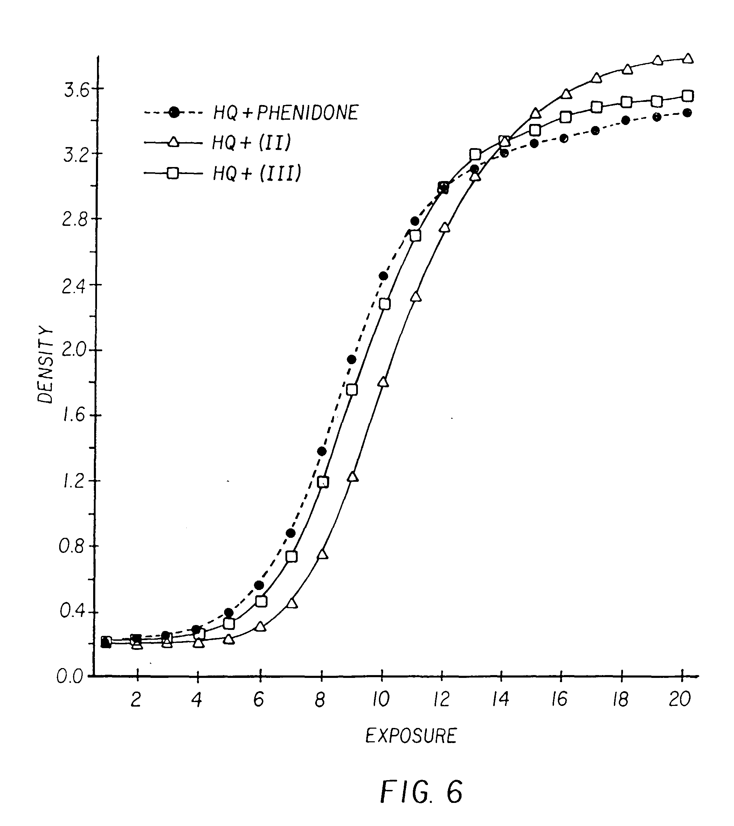

It can be seen in Figure 6 that in "rapid access" mode compounds II or III associated with hydroquinone give a sensitometry very close to that obtained with hydroquinone combined with phenidone-A.

## Claims

1. Aqueous solution for the development of silver halide photographic products, said solution comprising (1) a silver halide developing agent selected from reductones or compounds of the hydroquinone type, and (2) at least one co-developing agent of formula: 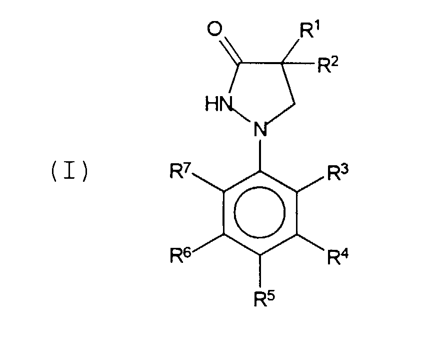 where R¹ and R² individually represent, alkyl, substituted alkyl, or a group represented by the formula:
(CH₂)ₘ― (L)ₙ ― A ― (Sol)
where
m is between 0 and 5 and n is 0 or 1,
L represents a divalent group selected from 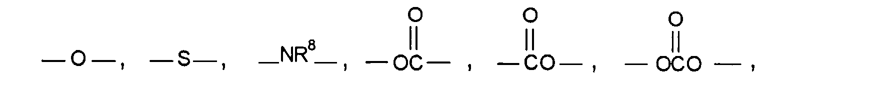 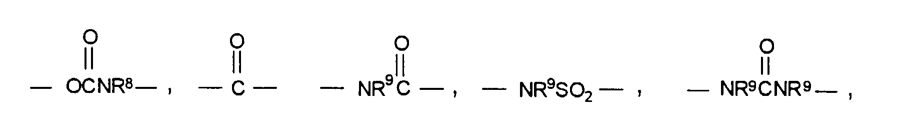 where R⁸ = R⁹ or A-(Sol), R⁹ = H, alkyl or aryl;
A represents a divalent group selected from 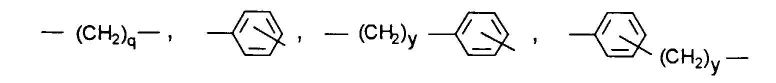 where q is between 0 and 5, and y is between 1 and 3;
(Sol) is a solubilising group selected from:
CO₂H, SO₃H, NHSO₂R¹⁰, SO₂NH₂, SO₂NHR¹⁰ polyhydroxyalkyl, 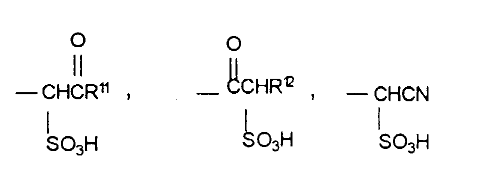 where R¹⁰ is alkyl or aryl, R¹¹ is OH, alkyl or aryl and R¹² is hydrogen, alkyl or aryl;
R³ to R⁷ in formula (I) individually represent hydrogen, alkyl, substituted alkyl, alkoxy, substituted alkoxy, aryloxy, substituted aryloxy, or a group represented by the formula:
(X)ₚ ― (CH₂)ₘ― (L)ₙ ― A ― (Sol)
where p = 0 or 1;
X represents a divalent group selected from -O-, -S-, -NR⁸- m, L, n, A, (Sol) and R⁸ are as defined previously with the additional proviso that at least one of the radicals R¹ to R⁷ must contain a group (Sol) and that (sol) is not directly attached to the phenyl ring or to the pyrazolidone ring.

2. Aqueous solution according to Claim 2 wherein the co-developing agent has the formula:

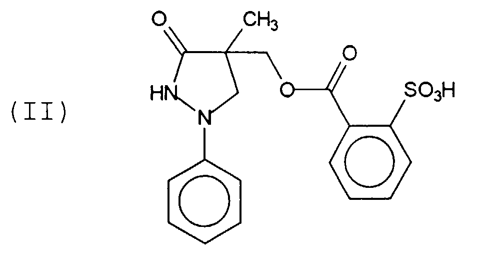

3. Aqueous solution according to Claim 1 wherein the co-developing agent has the formula:

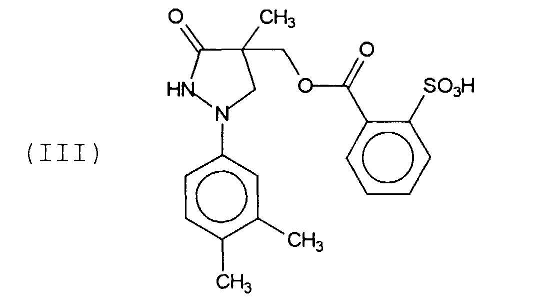

4. Aqueous solution according to Claim 1 wherein the co-developing agent has the formula:

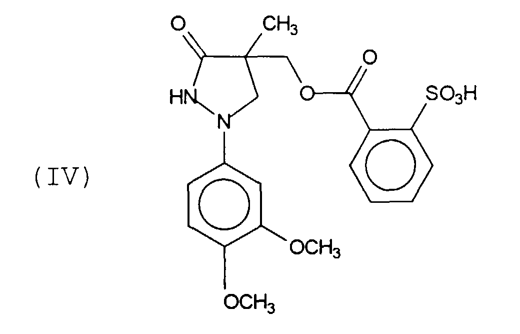

5. Aqueous solution according to any of Claims 1-4, in which the developing agent is selected from ascorbic acid, sugar-type derivatives of ascorbic acid, the stereoisomers, diastereoisomers and precursors of these acids and their salts.

6. Aqueous solution according to Claim 5 in which the developing agent is selected from ascorbic acid, D-isoascorbic acid, methyl-2-cetogluconate and 5,6-isopropylene ascorbic acid.

7. Photographic development process consisting of bringing an exposed photographic product into contact with the developing solution according to any one of Claims 1 to 6.

8. Process according to Claim 7, in which the photographic product is brought into contact with the developing solution for less than 1 minute.

## Patentansprüche

1. Wässrige Lösung zur Entwicklung von fotografischen Produkten auf Silberhalogenidbasis, **dadurch gekennzeichnet,** dass die Lösung (1) einen Silberhalogenidentwickler aus dem Bereich der Reduktone oder der Verbindungen vom Hydrochinon-Typ aufweist und (2) mindestens einen Co-Entwickler der Formel: 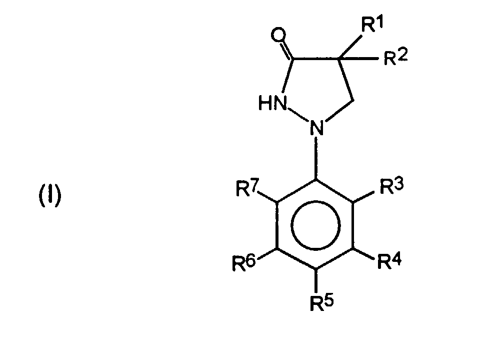 wobei R¹ und R² unabhängig voneinander Alkyl, substituiertes Alkyl oder eine Gruppe der Formel:
(CH₂)ₘ - (L)ₙ - A - (Sol)
sind, in der m zwischen 0 und 5 variiert und n 0 oder 1 ist,
wobei L eine der folgenden zweiwertigen Gruppen ist: 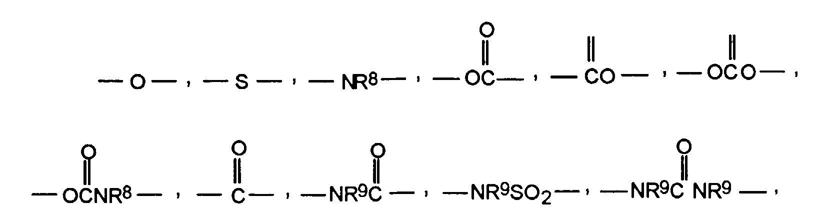 in denen R⁸ = R⁹ oder A - (Sol), R⁹ = H, Alkyl oder Aryl ist;
wobei A eine der folgenden zweiwertigen Gruppen ist: 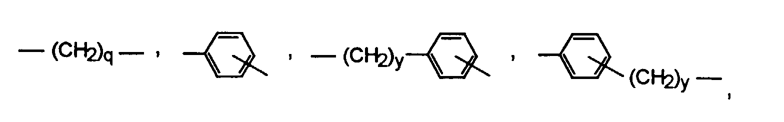 in denen q zwischen 0 und 5 variiert und y zwischen 1 und 3;
wobei (Sol) eine der folgenden solubilisierenden Gruppen ist:
CO₂H, SO₃H, NHSO₂R¹⁰, SO₂NH₂, SO₂NHR¹⁰, Polyhydroxyalkyl, 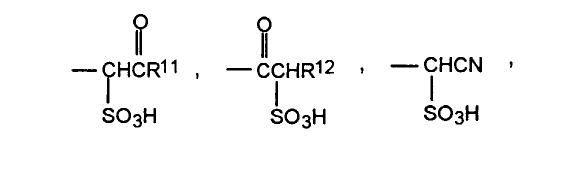 in denen R¹⁰ Alkyl oder Aryl, R¹¹ OH, Alkyl oder Aryl und R¹² Wasserstoff, Alkyl oder Aryl ist;
wobei R³ bis R⁷ in Formel (I) unabhängig voneinander Wasserstoff, Alkyl, substituiertes Alkyl, Alkoxy, substituiertes Alkoxy, Aryloxy, substituiertes Aryloxy oder eine Gruppe der Formel ist:
(X)ₚ―(CH₂)ₘ―(L)ₙ―A―(Sol)
in der p = 0 oder 1;
wobei X eine der zweiwertigen Gruppen -O-, -S-, -NR⁸- ist, und
wobei m, L, n, A, (Sol) und R⁸ die gleiche Bedeutung wie zuvor haben, mit dem zusätzlichen Vorbehalt, dass mindestens einer der Reste R¹ bis R⁷ eine Gruppe (Sol) aufweisen muss und (Sol) nicht direkt an den Phenyl-Ring oder den Pyrazolidon-Ring gebunden ist.

2. Wässrige Lösung nach Anspruch 1, dadurch gekennzeichnet, dass der Co-Entwickler folgende Formel hat:

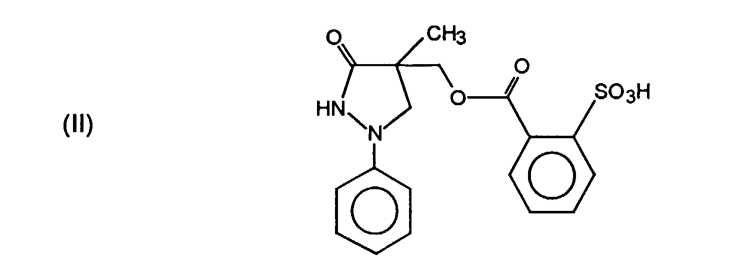

3. Wässrige Lösung nach Anspruch 1, dadurch gekennzeichnet, dass der Co-Entwickler folgende Formel hat:

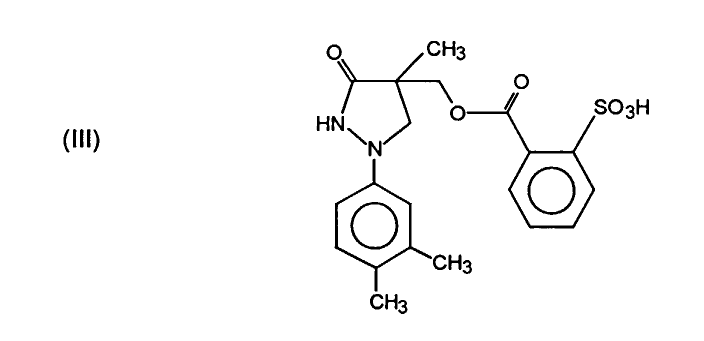

4. Wässrige Lösung nach Anspruch 1, dadurch gekennzeichnet, dass der Co-Entwickler folgende Formel hat:

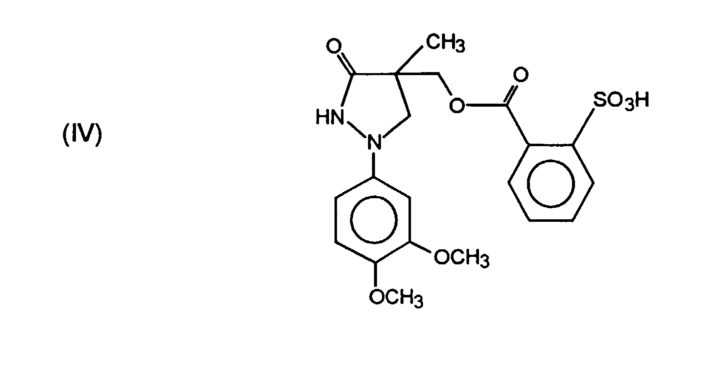

5. Wässrige Lösung nach einem der Ansprüche 1 - 4, dadurch gekennzeichnet, dass der Entwickler aus der Gruppe Ascorbinsäure, zuckerartige Derivate der Ascorbinsäure, den Stereoisomeren, Diastereoisomeren und Vorgängerverbindungen dieser Säuren und ihrer Salze ausgewählt wird.

6. Wässrige Lösung nach Anspruch 5, dadurch gekennzeichnet, dass der Entwickler aus der Gruppe Ascorbinsäure, D-lsoascorbinsäure, Methyl-2-cetogluconat und 5,6-lsopropylidenascorbinsäure ausgewählt wird.

7. Fotografisches Entwicklungsverfahren, dadurch gekennzeichnet, dass ein belichtetes fotografisches Produkt in Kontakt mit der Entwicklerlösung nach Anspruch 1 bis 6 gebracht wird.

8. Fotografisches Entwicklungsverfahren nach Anspruch 7, dadurch gekennzeichnet, dass das fotografische Produkt für weniger als eine Minute mit der Entwicklerlösung in Kontakt gebracht wird.

## Revendications

1. Solution aqueuse pour le développement de produits photographiques aux halogénures d'argent comprenant (1) un développateur des halogénures d'argent choisi parmi les réductones ou les composés du type hydroquinone et (2) au moins un co-développateur de formule : 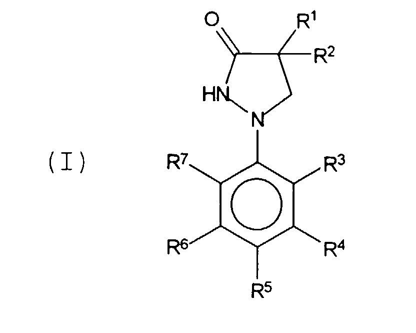 où R¹ et R² chacun séparément représentent un groupe alkyle substitué ou non, ou un groupe représenté par la formule :
(CH₂)ₘ―(L)ₙ―A―(Sol)
où m est de 0 à 5 et n est 0 ou 1,
L représente un groupe divalent choisi parmi 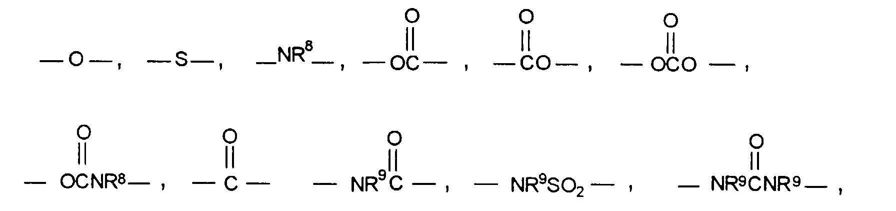 où R⁸ = R⁹ ou A-(Sol), R⁹ = H, alkyle ou aryle ;
A représente un groupe divalent choisi parmi 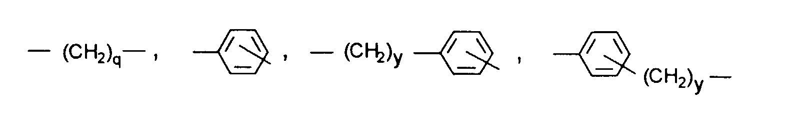 où q est compris entre 0 et 5, et y est compris entre 1 et 3 ;
(Sol) est un groupe solubilisant choisi parmi :
CO₂H, SO₃H, SO₂NH₂, NHSO₂R¹⁰, SO₂NHR¹⁰, polyhydroxyalkyl, 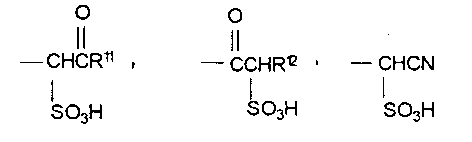 où R¹⁰ est alkyle ou aryle, R¹¹ est OH, alkyle ou aryle et R¹² est l'hydrogène, alkyle ou aryle ;
R³ à R⁷ dans la formule (I) représentent chacun séparément l'hydrogène, un groupe alkyle , alkoxy substitué ou non, aryloxy substitué ou non ou un groupe représenté par la formule :
(X)ₚ―(CH₂)ₘ ― (L)ₙ ― A ― (Sol)
où p = 0 ou 1 ;
X représente un groupe divalent choisi parmi - O- , - S - , -NR⁸ -
m, L, n, A, (Sol) et R⁸ sont tels que définis précédemment
avec la condition supplémentaire qu'au moins l'un des radicaux R¹ à R⁷ doit contenir un groupe (Sol) et que (Sol) n'est pas rattaché directement au noyau phényle ou au noyau pyrazolidone.

2. Solution aqueuse selon la revendication 1 où le co-développateur a la formule :

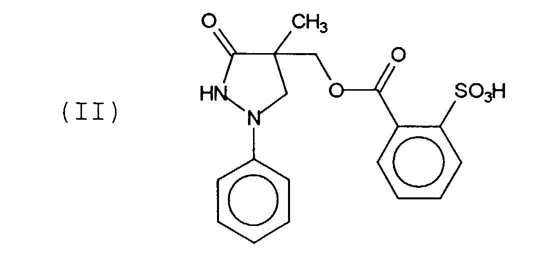

3. Solution aqueuse selon la revendication 1 où le co-développateur a la formule :

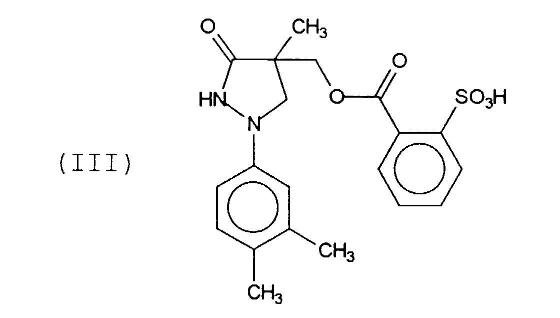

4. Solution aqueuse selon la revendication 1 où le co-développateur a la formule :

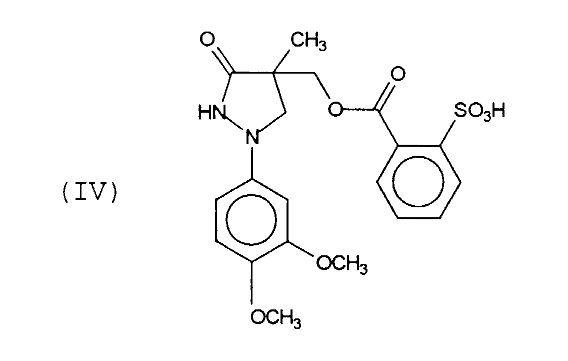

5. Solution aqueuse selon l'une des revendications 1 à 4, dans laquelle le développateur des halogénures d'argent est choisi parmi l'acide ascorbique, les dérivés du type sucre de l'acide ascorbique, les stéréoisomères, diastéréoisomères, précurseurs de ces acides et leurs sels.

6. Solution aqueuse selon la revendication 5 dans laquelle le développateur est choisi parmi l'acide ascorbique, l'acide D-isoascorbique, le méthyl-2-cétogluconate et l'acide 5,6-isopropylène ascorbique.

7. Procédé de développement photographique consistant à mettre en contact un produit photographique exposé avec une solution de développement selon l'une quelconque des revendications 1 à 6.

8. Procédé selon la revendication 7 dans lequel le produit photographique est mis en contact avec la solution de développement pendant moins de 1 minute.
